# EUROPEAN PATENT SPECIFICATION

(11) **EP 3 496 622 B1**
(45) Date of publication and mention of the grant of the patent: **08.09.2021**
(21) Application number: 17771830.1
(22) Date of filing: 14.08.2017
(51) Int. Cl.: A61B 10/02, A61J 15/00, G01N 33/487

(54) **ASPIRATORS WITH IN-LINE COLORIMETRIC TESTERS**
ABSAUGER MIT KOLORIMETRISCHEN INLINE-TESTERN
ASPIRATEURS À TESTEURS COLORIMÉTRIQUES EN LIGNE

(30) Priority: 12.08.2016 GB 201613893
(43) Date of publication of application: 19.06.2019
(73) Proprietor: Nasogastric Feeding Solutions Limited, Merseyside L3 5RF (GB)
(72) Inventor: GALLAGHER, George, Liverpool Merseyside L3 5RF (GB)
(74) Representative: Hutchinson, Thomas Owen
(86) International application number: PCT/GB2017/052390
(87) International publication number: WO 2018/029492

(56) References cited:
- EP-A1- 2 168 558
- WO-A1-90/03819
- CA-A- 803 255
- GB-A- 2 499 319
- US-A- 4 879 999
- US-A- 5 156 159
- US-A- 5 456 251
- US-A- 5 800 779
- US-A1- 2006 060 202
- US-A1- 2011 270 054
- US-A1- 2013 172 781
- US-A1- 2015 168 244

## Description

This invention relates to in-line testers, and in particular, but without limitation, to in-line testers that can be used in conjunction with a nasogastric (hereinafter "NG") aspirator to test for the correct placement of an NG tube.

Aspirators are used in a wide range of medical procedures where fluids need to be drawn from within a body cavity, for example, for diagnostic, sampling and/or therapeutic purposes.

An aspirator generally comprises a vacuum source connected to a tube that can be inserted, or fed, into a body cavity. The vacuum source can be of any suitable type, such as a syringe; a syringe-based manual pump (such as that disclosed in published UK patent No: GB2523591B); or an electric vacuum pump (such as that described in published UK patent application No GB2547012).

When the vacuum is applied to the free end of the tube, the vacuum draws fluid from the body cavity through the tube, provided, of course, that the tip of the tube is located within the fluid to be aspirated. A liquid trap is usually interposed between the tube and the vacuum source to prevent aspirated liquids from being drawn into the vacuum source, thereby reducing the likelihood of damaging and/or contaminating the vacuum source.

One example of a liquid trap is disclosed in published UK patent No: GB2523591B, in which a porous or perforated membrane is used to allow gasses to pass through it, but when wetted by aspirated liquids, blocks the aspirate from passing through it.

Other examples of relevant prior art include US 5 800 779 A, US 2006/060202 A1 and US 2011/270054 A1.

When using an aspirator, care needs to be taken to ensure that the vacuum is not too high and that the quantity and rate of aspiration is monitored. Monitoring and control circuitry can often be used to facilitate this, as well as the manual interventions of an experienced operator.

It is commonplace to use aspirators to assist in determining whether the tip of a NG feeding tube is correctly positioned. When feeding a patient using a NG feeding tube, care must be taken to ensure that the tip of the NG tube is positioned within the patient's stomach, rather than in his/her lungs. The reason that this is important is that a NG tube is fed into the patient's throat via his/her nose, and due to the bifurcation of the patient's throat into the oesophagus and trachea, it is possible for the tip of the NG tube to be fed into the trachea, rather than the oesophagus (i.e. the wrong way) resulting in the tip of the NG tube being positioned within the patient's lung. If feeding commences with the NG tube positioned in the patient's lung, the results can be very serious.

As such, medical protocols require that before NG feeding commences, correct positioning of the NG feeding tube tip is determined. The only definitive way to determine the correct placement of a NG feeding tube is via a chest X-ray or other imaging procedure. However, chest x-rays have been found, in certain cases, to be less than definitive because the angle of exposure, level of exposure, patient position and the skill of the radiographer are all important in achieving a diagnostic x-ray image that is fit for purpose. Further, the time taken for an x-ray to be booked, carried out, processed and reported can vary considerably - during which time a subject is denied feeding, the subject most likely being in a critical condition. Additionally, for neonates particularly, the subject will be exposed to radiation with the associated potentially negative consequences.

Another way to check for the correct positioning of an NG tube is to aspirate and test a sample of fluid via the NG tube prior to feeding. This is indeed the current clinical standard in the UK, whereby fluids are aspirated via the NG tube and are checked for acidity using pH paper. A colour change to indicate acidic pH is considered to be an indicator that the NG tube is in the correct location (it being assumed that the stomach contents are acidic, whereas lung fluids are not) and so feeding may commence.

If, however, a basic (alkali or pH > 5.5) pH reading is obtained, then the assumption is that the location of the tube is not in the stomach and the NG tube will be withdrawn and reinserted before a further pH test is carried out.

However, pH testing has been found to be unreliable insofar as it can yield false negative results, for example, if the patient is taking antacids to prevent reflux. In this case, irrespective of whether the NG tube is in the stomach or not, a basic or neutral pH will be indicated from any sample obtained using the current clinical standard.

Therefore, the current guidelines stipulate that if an acidic pH reading is obtained, then it is safe to commence feeding, whereas if a basic (alkali) pH reading is obtained, further investigation is required. The basic "rule of thumb" is therefore: acid = feed; alkali/neutral = do not feed.

However, current pH testing protocols fail to take into account the possibility of false positive results (i.e. acid, but not safe to feed), which can prove problematic to patient health, or fatal in extreme cases. False positives can occur when the NG tube tip is located in the lung of a patient, but where the patient is suffering from reflux resulting in some gastric content being inhaled/present in the lung. Clearly, in this case, it is possible for the pH of an aspirated sample to be acidic - indicating, according to current clinical protocols, that the NG tube tip is in the correct location, i.e. in the stomach, and so feeding will commence. However, in this example, the NG tube would not be correctly located, and the consequences of commencing feeding could be very serious.

It will be appreciated that the aforesaid protocols, devices and methods are not ideal, and a need therefore exists for an improved and/or an alternative protocol, device and/or method.

The invention is are set forth in the appended independent claim or claims. Preferred or optional features of the invention are set forth in the appended dependent claims.

The in-line tester is suitably used in conjunction with an NG aspirator, that is so say, having its inlet connectable, in use, to the outlet of an NG tube, and its outlet connectable, in use, to a vacuum source. Suitably, therefore, the inlet or outlet may comprise connectors for releasably connecting items thereto, such a "Luer lock" connectors, bayonet-type fittings, screw threads, push-fit connectors, being either male or female. Such a configuration suitably facilitates attaching and detaching items to the in-line tester.

Suitably, the inlet comprises a connector for connecting the inlet to the connector of enteral or NG tube, such as a female Luer or female Luer-lock connector. Suitably, the outlet comprises a connector suitable for connecting it to an enteral syringe, such as a male Luer, a male Luer-lock or an "ENFit" connector.

The liquid-stop device suitably comprises a porous of perforated disc, which is at least partially manufactured from, or coated with, a hydrophobic material; or which has a hydrophobic layer on it. The function of such a liquid-stop device is essentially that; when it is dry, gasses (including air) are able to pass through the pores or perforations therein, thus permitting the passage or transduction of gases/air through the liquid-stop device. However, when the liquid-stop device is wetted by a liquid, in this case an aspirated liquid, the hydrophobicity of the liquid-stop device repels the liquid from its surface - towards the pores/perforations, which are not hydrophobic. This results in liquid preferentially covering the liquid-stop device's pores/perforations, thereby preventing gas/air from passing through it. As such, when the liquid-stop device is dry, for example, during the initial stage of an aspiration, where air is primarily drawn up through the NG tube, the aspirated air is able to pass through the liquid-stop device. However, subsequently, when liquid is aspirated, when that liquid eventually reaches the liquid-stop device, it will wet it, thereby preventing any further passage of fluid (that is to say liquids or gases) through the liquid-stop device.

In one possible embodiment of the invention, the liquid-stop device comprises a porous or perforated element, which separates the inlet from the outlet at some point along the fluid flow path. Therefore, fluids (liquid or gases) drawn into the in-line tester must come into contact with the porous or perforated element at some point. In one possible embodiment, the liquid-stop device comprises a small piece of pH indicator paper with a hydrophobic plastics backing layer. Thus, liquids drawn into the in-line tester can be tested for pH by the pH paper, but when the pH paper is wetted, the liquid also wets the hydrophobic layer behind it, thereby closing-off the fluid pathway. In other embodiments, the pH paper can be substituted for capnometry indicator paper, which changes colour in the presence, or otherwise, of carbon dioxide.

The test trip (pH or CO2) and hydrophobic layer can be integrally formed, for example by laminating or bonding the test strip to the hydrophobic layer; in other cases, they can be separate components, which are simply placed together; or in yet further embodiments, they can be entirely separate, that is to say, with a finite gap, of any size, between the test strip or tester and the liquid-stop device.

The liquid-stop device inhibits or prevents the passage of fluids from the inlet to the outlet, thereby effectively acting as a valve, which automatically closes the fluid passageway between the inlet and the outlet upon coming into contact with a liquid.

This is particularly beneficial in NG aspirator applications, where gasses (air/gas from the stomach/lung) are usually aspirated before liquids. Thus, by placing the colorimetric capnometer downstream of the liquid-stop device and before the outlet, sequential testing may be possible. Specifically, when the in-line tester of the invention is used in conjunction with an NG aspirator, typically gasses will be aspirated before liquids are drawn-up through the NG tube. Initially, the liquid-stop device will be dry, which enables the aspirated gasses to pass through it to then come into contact with the colorimetric capnometer, and so test for carbon dioxide in the aspirate. Later, liquids may then be aspirated, and when this happens, the liquids come into contact with the first element, and are tested for the target substance thereby. When, eventually, the liquid-stop device is wetted by an aspirated liquid, it inhibits or prevents the passage of liquids through it, thereby keeping the colorimetric capnometer dry.

Suitably, a one-way valve is provided at, or downstream of the outlet of the in-line tester, and where such is provided, a sample of aspirated gas can be trapped/retained within the chamber between the (now wetted) liquid-stop device and the one-way valve. This is particularly beneficial when using colorimetric capnometry because colorimetric capnometers tend to revert to their initial colour after a relatively short period of time. However, by effectively trapping a sample of aspirated gas in the chamber between the liquid-stop device and a one-way check valve, reversion of the colour of the colorimetric capnometer back to its initial state is slowed or inhibited.

The first tester is adapted to exhibit a colour change in the presence of a target substance. The target substance is suitably a substance found in the stomach of a patient.

The target substance can be stomach acid (e.g. HCI), in which case, the first tester may comprise, e.g. on a liquid-absorbent layer thereof, a colorimetric substance that is an acid-base indicator, such as litmus paper or paper comprising Bromothymol sulfonephthalein.

Additionally or alternatively, the target substance can be a stomach-related marker, which may comprise any compound or biological structure, such as a cell or a cell fragment, an enzyme, a chemical etc., which is found within the stomach of a patient, but preferably not the lung of a patient.

In certain embodiments of the invention, the stomach-related marker may comprise any one or more of the group comprising: gastric enzyme (or substrate thereof); gastric hormone; pepsin; pepsinogen; intrinsic factor (IF); vitamin B12-IF complex; mucin; gastrin; gastric lipase; and trypsin.

In an embodiment, the in-line tester comprises detection means for gastric lipase. The detection means for gastric lipase may comprise tributyrin.

The device may contain means for detecting the presence of two or more stomach-related compounds. Advantageously, the device comprises means for detecting two stomach-related markers.

Suitably, therefore, the in-line tester may comprise a further tester having a further colorimetric substance adapted to exhibit a colour change in the presence of a second target substance. In certain embodiments, the first and further testers may be incorporated into a single device, that is to say being is divided into discrete regions, each discrete region comprising a different colorimetric substance adapted to exhibit a colour change in the presence of different target substances.

Suitably, the outlet of the porous or perforated element sealingly separates the inlet from the outlet.

Suitably, the liquid-stop device comprises a hydrophobic layer that comprises pores or perforations that enable, when dry, air to pass through them, but when wetted by liquid, the hydrophobicity of the hydrophobic layer repels the liquid from its surface and is forced towards the openings of the pores or perforations.

The colorimetric capnometer is adapted to detect carbon dioxide, and this may be by using a specially adapted form of indicator paper, impregnated with a dye that changes colour from, say, purple to yellow in the presence of carbon dioxide. Carbon dioxide monitoring to check NG tube position has been suggested (Thomas and Falcone, J Am Coll Nutr 1998, 17(2):195-7). Various trials have used either capnography (direct carbon dioxide measurement) or colorimetric capnometry (based on colour change of adapted pH paper with sulfonephthalein). It has already been shown that that there is a higher concentration of carbon dioxide in exhaled air from the lungs compared to any air obtained from a gastric aspirate. However, the use of measuring carbon dioxide provides no information about tube placement within the gastrointestinal tract and administration of enteral nutrition may be delivered into the oesophagus which would increase the risk of aspiration to the lung.

The Applicants have identified the problems associated with the prior art and surprisingly discovered that a combination of being able to detect at least one stomach-related marker and carbon dioxide provides a much more reliable means for determining the location of, for example, a NG feeding tube in a subject.

The detection means for carbon dioxide and at least one detection means for a stomach-related marker may be disposed on at least one substrate.

The substrate may comprise a matrix. Advantageously, the substrate comprises a cellulose-based matrix. The substrate may be porous and/or perforated to permit the flow of fluid therethrough.

In an embodiment, the device comprises two or more detection means for a stomach-related marker. The two or more detection means may be disposed on the same substrate or different substrates.

The substrate may be an adapted form of pH filter paper, impregnated with a dye which changes colour from purple to yellow in the presence of carbon dioxide. Alternatively, the substrate may comprise adapted pH paper carrying sulfonephthalein or Bromothymol sulfonephthalein, which is an acid-base indicator.

The detection means for carbon dioxide may be capable of distinguishing the level of carbon dioxide present. A known carbon dioxide detector (available form Mercury Medicalhttp://www.mercurymed.com/catalogs/ADR_CarbonDioxideDetector.pdf) changes colour depending upon the level of carbon dioxide present. 5% carbon dioxide detected in a sample is indicative of normal exhalation value and would indicate that the NG tube is located in the lung of a subject. Levels below that would indicate that the NG tube is either not in the lung or that the subject may be experiencing other medical problems, particularly where a stomach-related marker is not detected, indicating that the tube may possibly be in the lung.

The subject may be a mammal. Advantageously, the subject is a human.

Preferred embodiments of the invention shall now be described, by way of example only, with reference to the accompanying examples and drawings in which:
Figure 1 is a perspective view of first embodiment of an in-line tester in accordance with the invention;
Figure 2 is an exploded view of the in-line tester of Figure 1;
Figure 3 is a sectioned view of the in-line tester of Figure 1;
Figures 4 and 5 are plan views of decals suitable for use with the in-line tester of Figure 1;
Figure 6 is a schematic plan view of a second embodiment of an in-line tester in accordance with the invention;
Figure 7 is a schematic sectional view of Figure 6 on VII-VII;
Figure 8 is a schematic sectional view of Figure 6 on VIII-VIII;
Figure 9 is a schematic sectional view of Figure 6 on IX-IX;
Figure 10 is an exploded view of Figure 9;
Figure 11 is a schematic circuit diagram of the embodiment of the in-line tester of Figures 1 to 3;
Figure 12 is a schematic circuit diagram of the embodiment of the in-line tester of Figures 6 to 9;
Figure 13 is a schematic exterior view of the in-line tester shown in Figure 6; and
Figure 14 is a schematic circuit diagram showing a variation of the circuit of Figure 12, incorporating a coarse liquid-stop device.

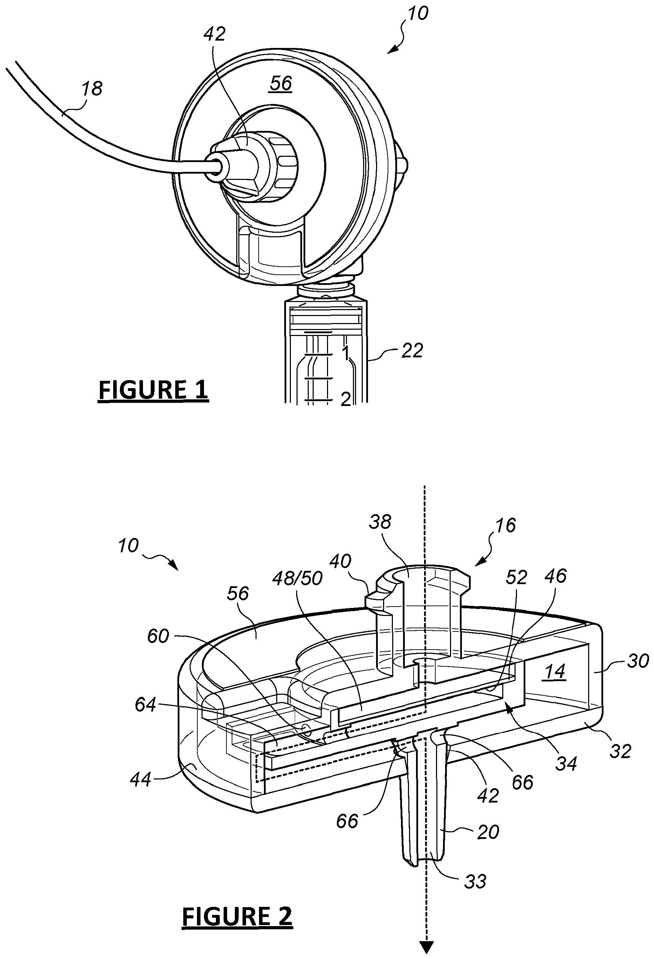

Referring to Figures 1 to 5 of the drawings, the in-line tester 10 comprises a main body 12 manufactured via a plastics injection moulding process from a transparent material, such as ABS. The main body 12 defines a hollow interior chamber 14 and has an inlet 16 connectable, in use, to an NG tube 18, and an outlet 20, connectable in use to a vacuum source (e.g. a vacuum pump; or a syringe 22 - as shown in Figure 1). A vacuum is applied to the outlet 20 of the in-line tester 10 to draw a sample of aspirate (gas and/or liquid) from within a patient via the NG tube 18, and the aspirate enters the hollow interior chamber 14 of the in-line tester 10 via the inlet 16.

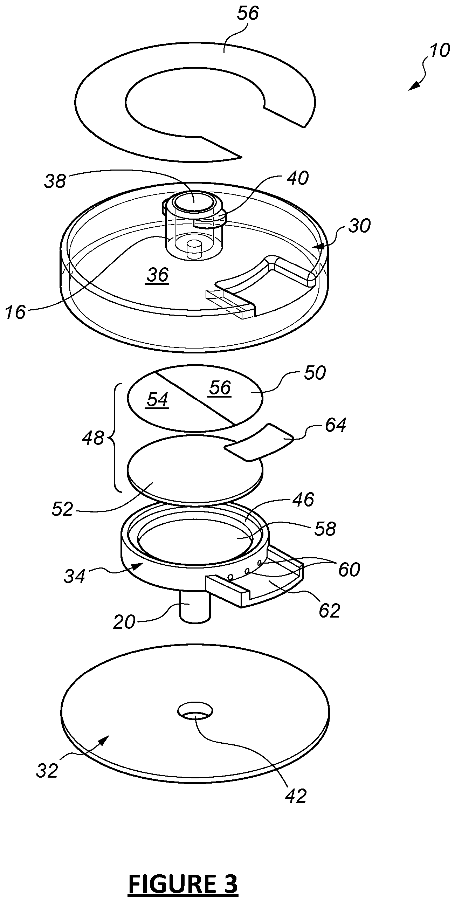

Referring to Figures 2 and 3 in particular, the main body 12 is formed by three main parts, namely: a generally dish-shaped first part 30; an annular back plate disc 32; and an insert 34.

The inlet 16 is formed as a tubular spigot extending concentrically from the outer face 36 of the generally dish-shaped first part 30. The inlet 16 has a through hole 38 that provides a fluid communication pathway into the interior of the main body 12, and also has external screw thread formations 40 for engaging complementary internal thread formations of a Luer-type connector 42 at the end of the NG tube 18.

The insert 34 is mostly located within the generally dish-shaped first part 30, but has an outlet spigot 20 formed integrally therewith, which sealingly extends through a tapered central through hole 42 in the annular back plate disc 32. The outlet spigot has a blind hole 33 in it (explained in greater detail below) and a plain outer surface, which can connect to the inlet of a syringe 22, or to a vacuum pump (not shown).

As can be seen by comparing Figures 2 and 3, the generally dish-shaped first part 30 has a planar peripheral edge 44, which is sealingly connected (for example by gluing or welding) to the outer periphery of the annular back plate disc 32. The insert 34 is thus retained in-situ.

The insert 34 has a generally circular dish-shaped profile, with an internally-rebated lip 46, which retains, by frictional engagement, a circular porous or perforated element 48 (first tester incorporating a liquid-stop device). The dimensions of the lip 46 are sized so as to form a valve seat against which the porous or perforated element sealingly seats. A seal can be perfected, if necessary, using a bead of sealant or adhesive (not shown).

As can be seen from the drawings, the porous or perforated element 48 comprises an airpermeable membrane, which permits air to pass through it, but not fluids. In the illustrated embodiment, the porous or perforated element 48 is both a pH tester and a liquid-stop device, and thus comprises two components, namely a liquid-absorbent layer 50, such as paper (located closest to the inlet 16), and a porous or perforated hydrophobic layer 52 (downstream of the liquid-absorbent layer 50). The two layers 50, 52 are conjoined to form a laminated structure, although they may equally be just clamped or otherwise held together. The main advantage of putting the two layers in close proximity, or touching one another, is that the wetting of the liquid-absorbent layer 50 very quickly, if not immediately, also wets the liquid-stop device, i.e. porous or perforated hydrophobic layer 52. Thus, the wetting of the first layer 50 causes the liquid-stop layer 52 to automatically close off almost immediately. However, in other embodiments, there may be a separation between these two layers 50, 52, or indeed, they may be located in entirely different regions of the in-line tester 10.

In this embodiment, the liquid-stop device, i.e. the hydrophobic layer 52, comprises pores or perforations that enable, when dry, air to pass through them (i.e. through the hydrophobic layer 52). However, when wetted by liquid, e.g. liquid absorbed by the liquid-absorbent layer 50, the liquid is repelled from the surface of the hydrophobic layer 52 and forced to overlie the less hydrophobic regions, that is to say, the openings of the pores or perforations. Provided the pores or perforations of the hydrophobic layer 52 are small enough (i.e. significantly smaller than the size of a liquid droplet), the liquid that overlies the pores or perforations effectively blocks the pores or perforations, thus inhibiting or preventing the passage of air or liquid through them.

Most preferably, the size of the pores and/or perforations is selected to permit the passage of vapour through the liquid-stop device, but not larger liquid drops. The reason for this is that certain CO2 test papers require the CO2 to be a "wet sample" in order create the reaction that causes a colour change. Thus, many CO2 test papers are configured to detect CO2 in a "breath" sample, that is to say, a gas sample comprising CO2, plus a small amount of water vapour. This, the liquid-stop device of the invention is suitably configured such that water vapour (such as that found in a patient's breath sample) can pass therethrough, but not liquid droplets above a certain size, as would be the case where an actual liquid sample (e.g. saline, liquid water, liquid feed, stomach acid, bile etc.) has been aspirated.

The aforesaid configuration conveniently converts the in-line tester 10 into a self-closing valve that permits air or gasses (including, in certain cases, water vapour) to pass through it when the porous or perforated element 48 is dry, but which when the porous or perforated element 48 is wetted, selfseals to prevent fluids from passing through it and further downstream.

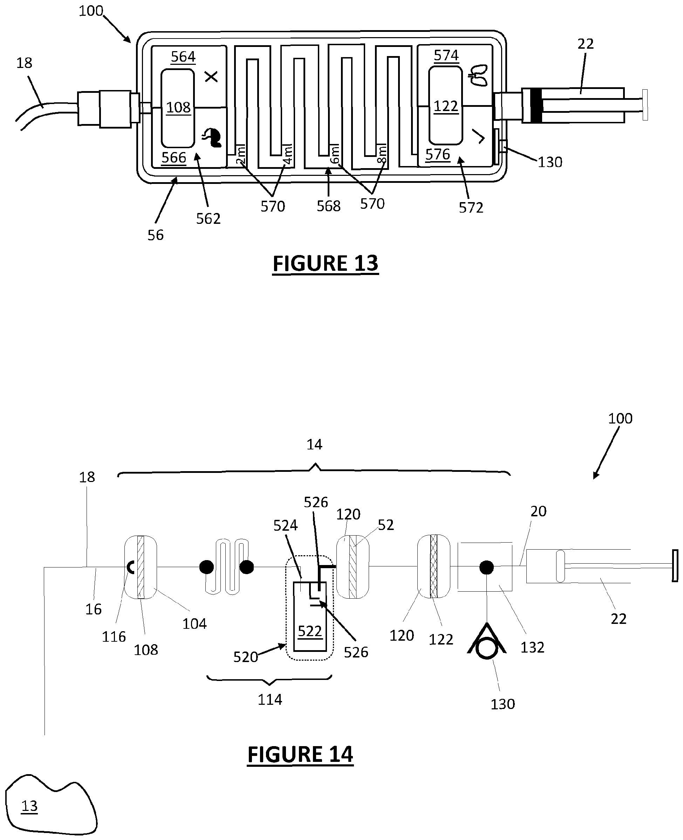

It will be appreciated that once the liquid-stop device has been wetted-out by an aspirated liquid sample, the device hydraulically locks, thereby preventing further aspiration of liquid or gas. Therefore, it is preferable that the liquid-stop device comprises a "coarse filter" and a "fine filter", the latter being, in many cases, the porous or perforated, hydrophobic membrane/element/disc described herein. The coarse liquid stop device could be a baffle arrangement, or a tortuous fluid pathway, which prevents or inhibits the fine filter from being inadvertently splashed with aspirated liquid. A detailed description of this is provided hereinbelow, with reference to Figure 14 of the accompanying drawings.

The liquid-absorbent layer 50 is coated or impregnated, in the illustrated embodiment, in two discrete areas 54, 56, by different colorimetric substances adapted to exhibit a colour change in the presence of different target substances.

In one embodiment, the first area 54 is adapted to change colour according to the pH of an aspirated liquid, and the second area 56 is adapted to change colour in the presence of a target stomach-related marker, as described herein. The colour of the two regions 54, 56 can be viewed from without the tester 10 via the transparent generally dish-shaped first part 30. A generally C-shaped decal 56, comprising a colour chart corresponding to the or each colorimetric substance is affixed to the outer surface 36 of the generally dish-shaped first part 30. In the illustrated embodiment, the generally C-shaped decal 56 surrounds, and slightly overlaps, the porous or perforated element 48 so that a visual comparison of colour of the porous or perforated element 48 to the decal 56 can be made.

In one embodiment, the liquid-absorbent layer is manufactured of litmus paper, and this forms the first area 54, such that the acidity/alkalinity of the aspirate can be tested. However, the second area 56 is a stomach-related marker detector, which is coated, or impregnated, with tributyrin, which tests for gastric lipase. Tributyrin will produce alcohol and butyric acid on contact with gastric lipase, and the butyric acid will lower the pH on the litmus paper, giving an acidic pH reading. This method can effectively correct the "false negatives" of relatively high pH in gastric aspirates from patients on antacids.

Below (downstream of) the porous or perforated element 48, the insert 36 comprises an internal chamber 58 into which aspirated gasses are vented after having passed through the porous or perforated element 48. The side wall of the insert 34 comprises one or more through apertures 60, through which the aspirated gasses are vented, in use. The apertures are located slightly above a shelf part 62 of the insert 34, upon which is located a colorimetric capnometer 64 in the form of a paper test strip impregnated with a substance that undergoes a colour change in the presence of greater than 5% CO2. The shelf part 62 has a base 63 and two side walls 65, which serve to frictionally retain the colorimetric capnometer test strip 64 when the device is assembled. This particular configuration usefully causes the aspirated gasses to be concentrated, and to vent over the surface of, the colorimetric capnometer test strip 64, thus enabling the colorimetric capnometer 64 to test more effectively for the presence of relatively low concentrations of CO2 in the aspirated gas.

The colorimetric capnometer test strip 64 comprises a dye carrying substrate (such as used in Mercury Medical carbon dioxide detector http://www.mercurymed.com/catalogs/ADR_CarbonDioxideDetector.pdf), which functions by detecting acid formed in exhalation of carbon dioxide form a subject. Indicator colour is indicative of the following conditions: Blue - No CO2 is present; Green - Between 1% - 2% CO2 is present; Yellow 5% or more CO2 is present.

The aspirated gas then flows out of the in-line tester 10, via the outlet 20, and this is accomplished by the provision of through holes 66 extending through the side wall of the outlet spigot 20 upstream of the annular back plate disc 32.

The flow path of aspirate is indicated schematically, in Figure 3, by the chain-dash arrow 70, that is to say, in via the inlet 16, through the porous or perforated element, out through the apertures 60 and over the colorimetric capnometer 64, under the insert 34, through holes 66 and out through the outlet 33, 20.

In use, the inlet 16 is connected to an NG tube 18 and an aspirate is drawn up the NG tube 18 and into the device 10. Initially, gas containing carbon dioxide will flow form the patient into the device, passing through the porous pH indicator 260 substrate, through the bores 223 and contact the carbon dioxide detecting substrate 280. If the carbon dioxide level is above a predetermined threshold to indicate exhaled air, then a colour change occurs in the carbon dioxide detecting substrate which if positive is indicative of the NG tube being located in the lung of a patient.

Subsequent aspiration results in liquid entering the device which is absorbed by liquid absorbent layer. If stomach acid is present, the pH detector will change colour to indicate the presence of acid, and if stomach enzyme or another target stomach-related substance is present, then an acid will be catalysed causing a pH indicator to show the presence of an acid.

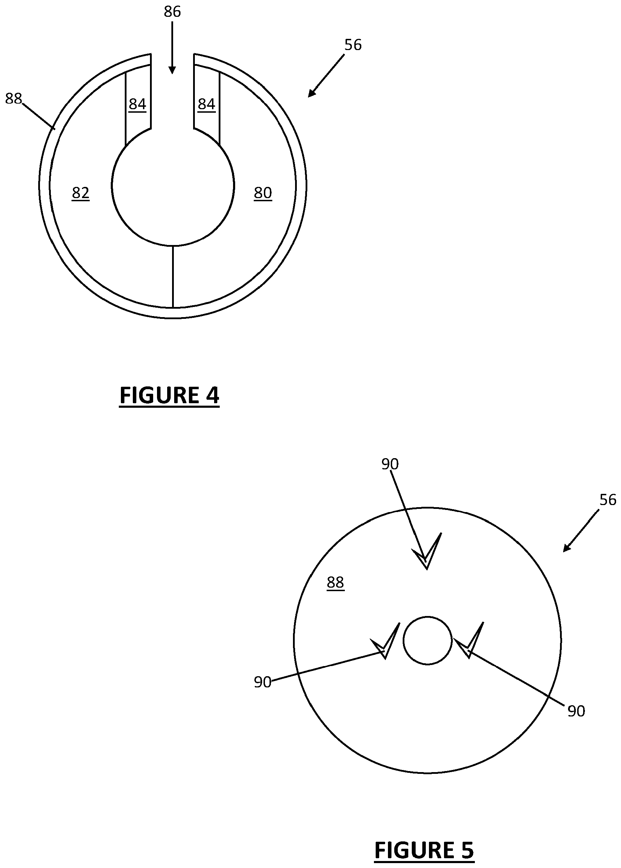

The decal 56 referred to previously, has different colour comparison areas, as shown in Figure 4 of the drawings, against which the respective colorimetric test strips can be compared, in use, by a medical practitioner. In the example shown in Figure 4, there are four areas. A first area 80 comprises a colour chart corresponding to the first area 54 of the porous or perforated element 48; a second area 82 comprises a colour chart corresponding to the second area 56 of the porous or perforated element 48; and a third area 84 (located either side of a cut-out 86) corresponding to the colorimetric capnometer. Thus, a practitioner can compare each of the three colorimetric test strips/areas against their corresponding colour charts. An outer peripheral region 88 is also provided, for displaying text, logos, instructions, CE markings etc..

In an alternative embodiment, as shown in Figure 5, the decal 56 is adapted to cover most/all of the upper surface of the main body. In this embodiment, its colour corresponds to a "fail" colour for each of the tests (which may be a different colour in different areas of the decal 56). The decal 56 has tick-shaped through apertures cut into it, such that if a "positive" test result is confirmed using each of the thee colorimetric tests, the respective test trip/area will become visible, due to a difference in colour, compared with the regions of the decal 56 surrounding each cut out 90.

The aforedescribed embodiment of the invention has been found, in clinical trials, and tests, to provide a solution to one or more of the problems outlined in the introductory part of this disclosure, namely providing a double - or triple check for the correct positioning, or otherwise, of the tip of an aspirator tube in a patient. However, in certain situations, practice/protocols dictate other NG tube use methodologies. For example, in certain hospitals/environments, patients are intubated each time a feed is to be given. In this case, a clean, empty NG tube is inserted into the patient immediately prior to each feed, and after each feed, the NG tube is withdrawn and then discarded. In these situations, the NG tube is empty prior to aspiration of gases/liquids, in which case the aforedescribed embodiment of the invention has been shown to work satisfactorily.

In other hospitals/environments, however, the feed protocols can be somewhat different. By way of an example, in certain hospitals, where a patient is receiving ongoing nasogastric feeding, the NG tube is kept in place and is only withdrawn/replaced at certain intervals. As such, the same NG tube may be used to feed a patient several times before it is eventually discarded and replaced. In these situations, except for the first time the NG tube is used, the NG tube will inevitably contain some liquid - be that stomach liquid, residual feed from the previous feed, or a saline flush solution. As such, when the NG tube is used subsequently and an aspirate taken, the first few drops of aspirate may not be representative of the liquid surrounding the tip of the NG tube, but rather may be representative of the residual contents of the NG tube. When the previously-described embodiment of the invention is used, in these situations, it can result in the aspiration test procedure being stopped prematurely, for example when a drop of saline flush liquid, or residual feed from within the tube, is aspirated onto the pH test strip, which according to the afore described embodiment, would result in the in-line tester being "hydraulically locked" by the liquid-stop device becoming wet. As such, in these situations, the aforedescribed in-line tester may be somewhat ineffective because, by virtue of its self-closing feature, it is unable to test a sample of aspirate from the region surrounding the NG tube's tip, but rather simply tests an aspirate that was already in the NG tube prior to the test commencing. A need therefore exists for a further embodiment of the invention, which addresses this particular issue.

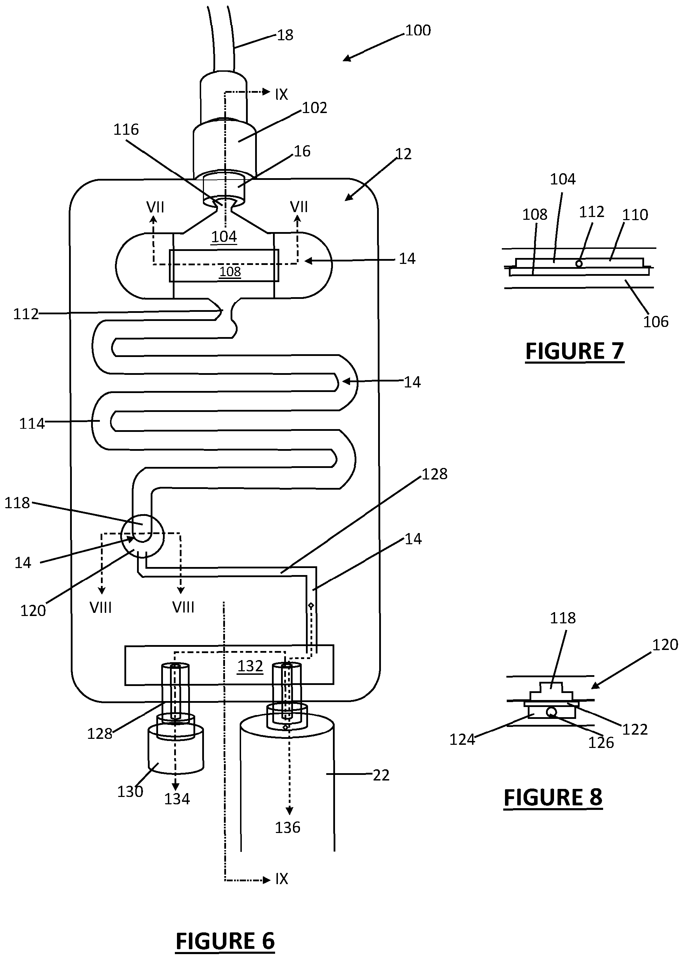

Referring now to Figures 6 to 10 of the drawings, another embodiment of an in-line tester 100 in accordance with the invention is shown. In this embodiment, the in-line tester 100 comprises a main body 12, again manufactured via a plastics injection moulding process, from a transparent material, such as ABS. The main body 12 defines a hollow interior chamber 14 as shall be described herein below.

The in-line tester has an inlet 16, which is connectable, in use, to an NG tube 18 via a luer connector 102. The inlet 16 connects the NG tube 18, to a first portion 104 of the chamber 14. The first portion 104 of the chamber 14, which is shown in cross section in Figure 7, comprises a base wall 106, which supports a small strip of pH indicator paper 108. A cavity 110 is provided on one side of the pH test strip 108, such that fluid entering the in-line tester 100, via the inlet 16 is able to come into contact with at least one surface or part thereof.

The first chamber part 104 has an outlet 112, which leads to a reservoir part 114 of the chamber 14.

The inlet 16 comprises a restriction, in the form of a Venturi 116 in a preferred embodiment, which causes liquid drawn up through the NG tube 18 to be "sprayed" over the exposed surface of the pH test strip 108. The provision of a constriction or Venturi 116 serves to cause the incoming liquid to "fan-out" as it enters the first chamber part 104, thereby ensuring that an adequate area of the pH test strip 108 is wetted by the incoming liquid.

The outlet 112 of the first chamber part 104 leads to a reservoir portion 114 of the chamber 14. In the illustrated embodiment, the reservoir 114 comprises a serpentine pathway within the main body 12, which can retain approximately 4ml of liquid, when full. The volume of the reservoir 114 is ideally selected to be slightly larger than the internal volume of the NG tube 18 and so the exact volume of the reservoir is not fixed. The reason for providing a reservoir 114 is to enable a quantity of liquid within the NG tube 18 to be accumulated within the main body 12 of the in-line tester 110, for reasons that shall become apparent later.

The reservoir part 114 of the chamber 14 comprises an outlet 118, which feeds into a further test chamber part 120 of the chamber 14. The further test chamber part 120 is shown in cross section in Figure 8 of the drawings, from which it can be seen that the outlet 118 of the reservoir is in fluid communication with a small disc tester 122 trapped, by its peripheries, between an upper part and a lower part of the main body 12. The test disc 122 is a CO2 tester, which exhibits a colour change in the presence of carbon dioxide.

The test disc 122 can be manufactured from a perforated, hydrophobic material, which enables gases exiting the reservoir 114 via its outlet 118 to pass through the test disc 122 and into a lower part 124 of the second test chamber 120, before exiting via an outlet 126 into a further pathway 128.

As gases are aspirated through the NG tube 18, they pass through the first chamber part 104, through the reservoir part 114 and eventually into the second test chamber 120, where they interact with the test disc 122. If the gas contains carbon dioxide, it will cause the test disc 122 to change colour, which will be visible to a user (not shown) observing the in-line tester 110, through its transparent main body portion. The aspirated gas can be drawn into a connected vacuum source, in the illustrated example, a syringe 22, and the aspirated gas can, therefore, be tested for the presence or otherwise of carbon dioxide.

In other embodiments of the invention, the test disc 122 is formed from two parts, namely a downstream colorimetric test disc 122, and an upstream porous or perforated element comprising a hydrophobic material 52. In this embodiment of the invention, the two parts work together as previously described, namely with the colorimetric test disc 122 being able to test for the presence of CO2, with the porous or perforated, hydrophobic layer 52, acting as a liquid-stop device 52 upstream of the colorimetric capnometer. Again, the two layers 122, 52 need not necessarily be in intimate contact with each other, although this may be beneficial in certain circumstances. Nevertheless, there is a liquid-stop device 52 located upstream of the CO2 tester, which means that when the test is complete, that is to say when a sample of aspirated liquid comes into contact with the porous or perforated, hydrophobic layer 52, the in-line tester 100 is essentially hydraulically locked, thereby preventing further aspiration. Meanwhile, a sample of aspirated gas will be trapped downstream of the liquid-stop device 52, thereby inhibiting or slowing the reversion of any colour change in the colorimetric capnometer for a certain period of time.

It is possible to connect the outlet of the in-line tester to a vacuum pump, or, in a preferred embodiment to a syringe 22.

Where a small syringe 22 is used, it may be necessary to repeatedly withdraw the syringe plunger (not shown) to obtain a sufficient test volume via the NG tube 18. To accomplish this, the inline tester 110 is provided with a secondary outlet 128, to which is connected a one-way valve 130. The syringe 22 and one-way valve 130 are operatively interconnected via a chamber 132 formed in the main body 12 of the in-line tester 100. The one-way valve 130 has a particular "cracking pressure", above which, the valve will open to allow gas to be expelled 134 from the in-line tester. The cracking pressure of the one-way valve 130 is designed to be lower than the permeability of the liquid-stop device such that upon depression of the syringe plunger (not shown) fluid is forced from the syringe 22, via the chamber 132 and out via the one-way check valve 130, as indicated by arrow 134 in Figure 6. However, upon withdrawing the plunger, the one-way check valve 130 closes, thus enabling fluid to be drawn up through the NG tube, through the in-line tester 100, and, ultimately, into the syringe, as indicated by arrow 136.

The benefits of this particular configuration of the invention are manifold.

In particular, because the main body 12 of the in-line tester 100 is manufactured from a transparent plastics material, it is possible for a user (now shown) to observe the progression of aspirated fluids through the tester 100. This usefully enables a user of the device to "see" when e.g. saline flush has been aspirated, followed by stomach content, for example, by a colour change in the reservoir.

In a first example, where the NG tube 18 is initially empty, upon repeated withdrawal and compression of the syringe 22 plunger, fluid will be "pumped" up through the NG tube in the manner previously described. Because the NG tube 18 is initially empty, the first fluid that will be drawn into the in-line tester 100 will be gas/air from within the NG tube itself or the patient's stomach/lung. This gas/air will simply flow through the first chamber part and over the pH test strip 108, through the reservoir 114, where it will eventually come into contact, and pass through the CO2 test disc 122.

The user, by observing the colour of the CO2 test disc 122 will be able to ascertain whether it is an air/stomach gas sample, or whether it is a "breath" sample of air aspirated from the patient's lung, for example.

Eventually, liquid (hopefully gastric juice) will be aspirated up the NG tube 18, where it will enter the in-line tester 100 via the inlet 16. The aspirated liquid will be "sprayed" by the Venturi 116 at the tester inlet 116, and the design of the Venturi 116 is such that aspirated liquid is sprayed/dispensed over the exposed surface of the pH test strip 108. By observing a colour change in the pH test strip 108, a user (not shown) will be able to determine whether an acidic, (e.g. a gastric juice) sample has been aspirated, or whether something else has been aspirated.

Liquid will then be drawn into the reservoir 114, where it will gradually fill the reservoir and the user will be able to observe the progress of the liquid as it is drawn into the reservoir.

In a second situation, for example where the NG tube 18 has been flushed with water or saline prior to use, it will initially contain a quantity, typically 4ml, of saline solution or water. In this situation, the "first liquid" to come into contact with the pH test strip 108 ought to be neutral, which might, ordinarily, indicate incorrect placement of the NG tubes tip in the patient's stomach. However, this could be a simple "false negative" because the first liquid aspirated is, in fact, saline solution rather than gastric juice. The test therefore needs to continue until such time as the flush liquid within the NG tube 18 has been recovered, which will, hopefully, be followed by a sample of gastric juice.

Therefore, the invention comprises a reservoir 114, into which this initial liquid may be accumulated. Here, the volume of the reservoir 114 is slightly greater than the internal volume of the NG tube 18 such that when the reservoir is full, the user knows that what is being tested ought to be gastric juice, rather than flush liquid. The user (not shown) is therefore able to observe the progression of the flush liquid through the system, by observing the reservoir, which is visible from outside the inline tester 100 by virtue of it being manufactured from a transparent plastic, and, ultimately, to test the pH of a gastric juice sample thereafter.

Although not shown in Figure 6 for clarity, the in-line tester 100 suitably comprises one or more decals, each comprising a colour chart corresponding to the or each colorimetric substance. The decal or decals are suitably affixed to an outer surface of the in-line tester 100, adjacent to, but preferably slightly overlapping the testers 108, 122 - so that a visual comparison of colour of the testers 108, 122 to the colours or other indications on the decal can be made.

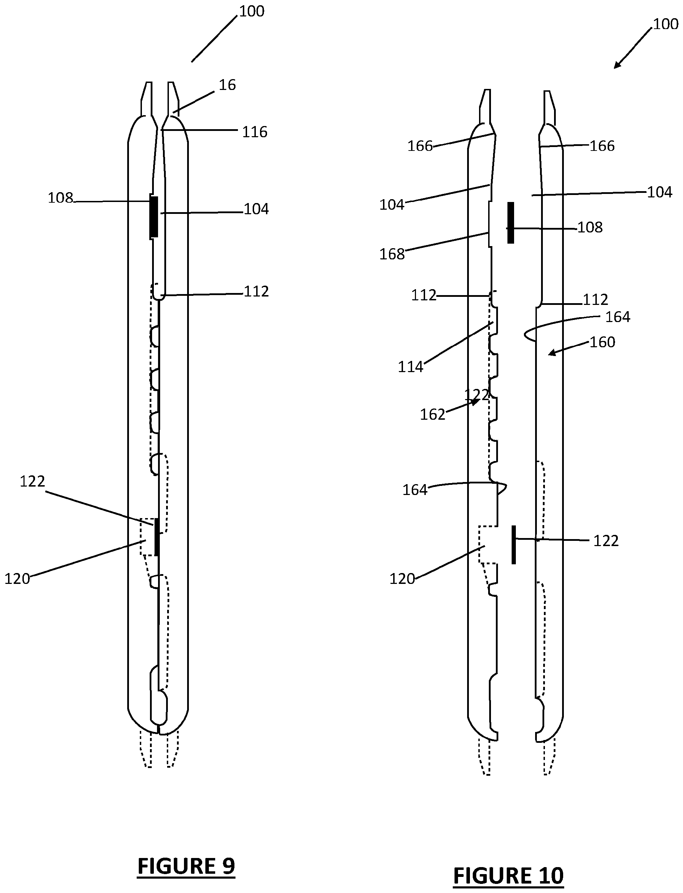

The in-line tester 100, shown in Figures 6, 7 and 8 of the drawings, is shown, schematically, in cross section in Figure 9, which is a cross section of Figure 6 on IX-IX.

In a preferred embodiment of the invention, the main body 12 of the in-line tester 100 is manufactured from two plastics injection moulded components, which fit together to form the device shown, schematically, in Figure 6. The various chambers 104, 120, 132 can be formed simply by providing recesses or grooves in the mating surfaces of the two components.

Referring to Figure 9 and 10 of the drawings - Figure 10 being an exploded view of Figure 9 the inlet 16 is formed in two halves from each of the main body pieces 160, 162. The two parts 160, 162 have a flat mating surface 164, which when pushed together, form a fluid-tight seal between the two pieces 160, 162. Channels or cavities within the in-line tester 100 can be formed by providing recesses or cavities in each of those mating surfaces 164.

Referring to Figures 9 and 10, it can be seen that the Venturi 116 is formed by a pair of opposing inclined surfaces 166 formed in each of the main body pieces 160, 162. The first cavity 104, which houses the pH test strip 108 is likewise formed with an additional recess part 168 formed in one of the pieces 162, for locating and retaining the pH test strip 108.

The outlet 112 of the first chamber part 104 is formed by complimentary recesses formed in each of the pieces 160, 162.

The serpentine reservoir 114 is formed by a correspondingly shaped serpentine groove formed in one of the main body pieces 162.

Likewise, the second chamber 120 is formed by a relatively deeper depression in the second main body part 162 and that enables the co2 test disc 122 to be housed therein. Further recesses formed in the main body pieces 160, 162 form the various other channels/cavities as indicated, schematically in Figures 9 and 10.

The two main body pieces 160, 162 can either be glued together, for example, by using an adhesive or welding along the shut line to form fluid-tight cavities/channels within the main body 12, or, where the mating surfaces 164 are sufficiently flat and/or intimate, such sealing may be accomplished by simply clamping, clipping, or otherwise holding together, the two main body pieces 160, 162.

By way of example, an in-line tester 100 similar to that described above with reference to Figures 6 to 10 of the drawings, is shown in Figure 13, in which a partially transparent decal 56 covers a front face of the in-line tester 100. The decal 56 has a first part 562, which surrounds the pH test strip 108 viewing window. The first part 562 is divided into two differently-coloured regions 564, 566, which are colored to match the colour of the pH paper 108 when a "fail" / "do not feed"; or a "pass" condition is detected, respectively. The two regions are indicated, for the avoidance of doubt by "X" and "stomach" indicia, respectively.

The reservoir 114 part of the in-line tester 100 is visible through a transparent 568 part of the decal 56. Graduations 570, indicating the volume of aspirated liquid, are optionally provided.

A third part 572 of the decal 56, which surrounds the CO2 test strip 122 viewing window. The third part 572 is divided into two differently-coloured regions 574, 576, which are colored to match the colour of the CO2 paper 122 when a "fail" / "do not feed"; or a "pass" condition is detected, respectively. The two regions 574, 576 are indicated, for the avoidance of doubt by "tick" and "lung" indicia, respectively.

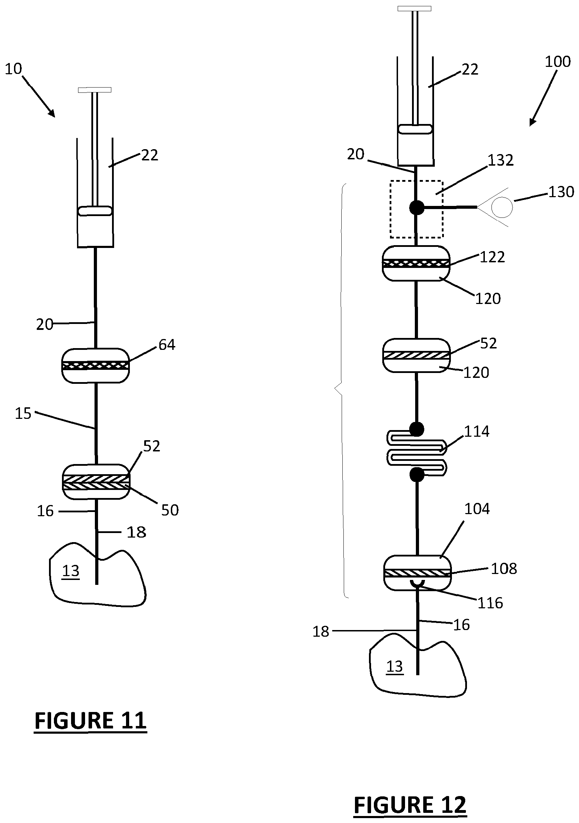

Referring now to Figures 11 and 12 of the drawings, which are schematic circuit diagrams for the in-line testers 10, 100 shown in Figures 1 to 3 and 6 to 9 above, respectively.

In Figure 11, the in-line tester 10 is fitted to an NG tube 18 at its inlet 16, and to a syringe 22 at its outlet 20. The tip of the NG tube is placed in the stomach 13 of a patient (not shown).

The in-line tester 10 comprises a chamber 14, which houses a first tester, namely a disc of pH test paper (e.g. litmus paper) 50, which is backed by a liquid-stop device, namely a perforated plastics, hydrophobic disc 52, which when wetted by aspirated liquids, closes-off and stops/inhibits further aspiration. A fluid passageway 15 connects the chamber 14 to a further chamber that houses a colorimetric capnometer 64, in this case, a strip of CO2-sensitive indicator paper that changes colour in the presence of CO2.

The syringe's plunger can be withdrawn to aspirate a sample of fluid from the stomach, via the NG tube 18 and into the in-line tester 10.

Aspirated gas passes through the pH test paper 50 and the liquid-stop device 52, where it then comes into contact with the colorimetric capnometer 64 to indicate the presence or otherwise of CO2 in the aspirated gas sample.

Thereafter, liquids may be aspirated from the stomach 13, via the NG tube 18, where they come into contact with the first tester 50 and indicate the presence, or not, of a target substance, e.g. stomach acid and/or a substance (e.g. a protein) only found in the stomach 13. The aspirated liquid contacts the liquid-stop device 52, causing the in-line tester 10 to hydraulically lock, thereby signifying the end of the procedure.

A one-way valve 130 may optionally be provided in a branch spurred-off between the syringe 22 and the outlet 20. This enables the syringe plunger to be depressed, and fluid within the syringe 22 to be vented via the one-way valve 132. This configuration permits a relatively small syringe 22 to be used as part of a pump, rather than having to use a relatively large syringe to obtain an adequate quantity of aspirate.

Referring now to Figure 12, the in-line tester 100 is fitted to an NG tube 18 at its inlet 16, and to a syringe 22 at its outlet 20. The tip of the NG tube is placed in the stomach 13 of a patient (not shown). The in-line tester 100 comprises a chamber 14, which has several parts.

A first chamber part 104 houses a first tester, for example a strip of pH test paper (e.g. litmus paper) 108 and/or a strip of other indicator paper, which changes colour in the presence of a target substance.

The first chamber part 104 is connected to a reservoir 114, which can accumulate a quantity of aspirated liquid.

The reservoir 114 connects to a further chamber 120, which houses a liquid-stop device 52, namely a perforated plastics, hydrophobic disc, which when wetted by aspirated liquids, closes-off and stops/inhibits further aspiration.

Downstream of the liquid-stop device 52, there is provided a colorimetric capnometer 122, in this case, a disc of CO2-sensitive indicator paper that changes colour in the presence of CO2.

A one-way valve 130 is provided in a branch spurred-off, e.g. via a chamber 132, between the syringe 22 and the outlet 20.

The syringe's plunger can be withdrawn to aspirate a sample of fluid from the stomach, via the NG tube 18 and into the in-line tester 10. Aspirated gas passes through the first tester 108 and the liquid-stop device 52, where it then comes into contact with the colorimetric capnometer 122 to indicate the presence or otherwise of CO2 in the aspirated gas sample.

Thereafter, liquids may be aspirated from the stomach 13, via the NG tube 18, where they come into contact with the first tester 104 and indicate the presence, or not, of a target substance, e.g. stomach acid and/or a substance (e.g. a protein) only found in the stomach 13. The aspirated liquid then fills the reservoir 114 until it eventually contacts the liquid-stop device 52, causing the inline tester 10 to hydraulically lock, thereby signifying the end of the procedure.

By virtue of the one-way valve 130, the syringe plunger to be depressed, and fluid within the syringe 22 can be vented via the chamber 132 and the one-way valve 132. This configuration permits a relatively small syringe 22 to be used as part of a pump, rather than having to use a relatively large syringe to obtain an adequate quantity of aspirate.

Referring now to Figure 14 of the drawings, a slight variation of the circuit diagram shown in Figure 12 is described. Identical reference signs have been used to identify identical features, for the avoidance of repetition, and for clarity. In Figure 14, it can be seen that the in-line tester 100 has been modified by the addition of a coarse liquid-stop device 520 locate upstream of the previously-described porous or perforated, hydrophobic membrane/disc 52. The coarse liquid-stop device 520 comprises a chamber 522, having an inlet 524 connected to the outlet of the reservoir 114, and an outlet 526 connected to the inlet of the chamber 120. Baffling 526 is provided within the chamber 522, to prevent liquid drops from being inadvertently splashed onto, or reaching the outlet 526. Here, liquids and gasses can be drawn into the coarse liquid-stop device 520, as may happen when the reservoir 114 is full, and the coarse liquid-stop device 520 provides a further means for preventing the porous or perforated, hydrophobic membrane/disc 52 from wetting out, as liquid droplets will be collected in the chamber 522, rather than passing through the coarse liquid-stop device 520 to the porous or perforated, hydrophobic membrane/disc 52 downstream of it.

The main purpose of the coarse liquid-stop device 520 is that it enables, where the reservoir 114 is full of aspirated liquid, say saline flush, to nevertheless permit the passage of air/gas bubbles to the CO2 test strip 122, via the porous or perforated, hydrophobic membrane/disc 52. This may occur where an NG tube has been used previously and thus contains a saline flush liquid. However, if, somehow, the NG tube has become misplaced, as may happen where the patient "wretches" the NG tube back up their oesophagus, then the next time the NG tube needs to be used, it will be checked for correct placement. Now, the first few ml of aspirate will be saline flush, or residual feed within the NG tube, and this liquid will fill, or partially fill the reservoir 114. Subsequent aspiration eventually empties the NG tube 18, such that gas is now, finally, aspirated. This aspirated gas will bubble through the liquid already in the reservoir, and without a coarse liquid-stop device 520 present, the liquid would tend to be splashed onto the porous or perforated, hydrophobic membrane/disc 52, thereby wetting it, and causing the in-line tester 100 to hydraulically lock. However, by placing a coarse liquid-stop device 520 upstream of the porous or perforated, hydrophobic membrane/disc 52, the aspirated gas is able to pass through the reservoir, and the porous or perforated, hydrophobic membrane/disc 52 before it reaches the CO2 paper 122, with the splashed liquid effectively being filtered-out by the coarse liquid-stop device 520. This enables a user to reliably test an aspirated gas sample - even after already having aspirated a liquid sample.

The coarse liquid-stop device 520, where provided, can be incorporated into the reservoir 114, or into the chamber 120, as desired.

### EXAMPLE 1:

Samples were used to generate the following truth table comparing a device in accordance with the present invention having means for pH detection, enzyme detection and carbon dioxide detection with the current clinical standard (pH paper).

There were three scenarios: 1) Normal pH content in stomach; 2) Patient on antacid medication; and 3) Stomach content in the lung. Each method and device was used and the results set out below.

**TABLE 1:**

| **SCENARIO** | **DEVICE IN ACCORDANCE WITH PRESENT INVENTION** | **CURRENT CLINICAL STANDARD (PH PAPER)** |
|---|---|---|
| Normal pH content in stomach | Confirmed | Confirmed |
| Patient on antacid medication | Confirmed | False negative confirmation tube not in stomach, patient has delayed feed |
| Stomach content in the lung | Confirmed | False negative confirmation tube not in stomach, patient has delayed feed |

### EXAMPLE 2:

Samples were used to generate the following truth table comparing a device in accordance with the present invention having means for pH detection, enzyme detection and carbon dioxide detection with the current clinical standard (pH paper). In this example, the three markers tested for in the device according to the present invention are separated out to give a clearer demonstration of the advantages of a device in accordance with the present invention.

There were three scenarios: 1) Normal pH content in stomach; 2) Patient on antacid medication; and 3) Stomach content in the lung. The presence or absence of three markers was known and each method and device was used and the results set out below.

**TABLE 2:**

| **SCENARIO** | **1^{ST} MARKER PH** | **2^{ND} MARKER ENZYME** | **3^{RD} MARKER CO₂** | **DEVICE ACCORDING TO PRESENT INVENTION** | **CURRENT CLINICAL STANDARD (PH)** |
|---|---|---|---|---|---|
| Normal pH content in stomach | Positive | Positive | Negative | 1^{st}, 2^{nd} and 3^{rd} markers confirmed | 1^{st} marker confirmed only |
| Patient on antacid medication | Negative | Positive | Negative | 1st, 2nd and 3rd markers confirmed | None or confirmed |
| Stomach content in the lung (or M. Cattarhalis infection in lung) | Positive | Positive | Positive | 1st, 2nd and 3rd markers confirmed | 1^{st} confirmed only - dangerous |

In complicated situations where for example, a patient is taking antacids or there is stomach content in the lung, only devices in accordance with the present invention will confirm the actual location of the NG tube for enteral feeding. Known devices and methods will only give an accurate indication of location when a patient has a normal stomach pH. In all other scenarios, the result from using known devices can be dangerous and has potentially fatal consequences should feeding via the tube be initiated where an incorrect placement of the tube is mistakenly indicated as being in the stomach.

The invention is not restricted to the details of the foregoing embodiments, which are merely exemplary of the invention which is defined by the appended claims. For example, any shapes, sizes, relative dimensions etc. are illustrative, and not limiting, as are any material selections and/or design choices (e.g. type of check valve).

## Claims

1. An in-line tester (10) comprising:
an inlet (38), connectable, in use, to an aspirator tube (18);
an outlet (33) connectable, in use, to a vacuum source; and
a chamber (14) interposed between the inlet (38) and the outlet (33), the chamber (14) comprising:
a first tester (48) comprising a first colorimetric substance adapted to exhibit a colour change in the presence of a target substance arranged such that fluids drawn into the chamber (14) by the vacuum source come into contact therewith;
the in-line tester (10) further comprising a liquid-stop device (52) which, when dry, permits the passage of gas from the inlet (38) to the outlet (33), but when wetted by a liquid, inhibits or prevents the passage of fluids from the inlet (38) to the outlet (33);
and being **characterised by**,
the chamber (14) further comprising
a second tester (64) comprising a colorimetric capnometer, which exhibits a colour change in the presence of carbon dioxide arranged such that fluids drawn into the chamber (14) by the vacuum source pass through it;
a reservoir interposed between the first tester(48) and the second tester (64);
the second tester (64) being located downstream of the liquid-stop device (52) and before the outlet (33).

2. The in-line tester (10) of claim 1, wherein the target substance comprises an acid, and wherein the colorimetric substance comprises an acid-base indicator.

3. The in-line tester (10) of claim 2, wherein the acid-base indicator comprises litmus paper, or bromothymol sulfonephthalein.

4. The in-line tester (10) of any preceding claim, wherein the target substance comprises a stomach-related marker being any one or more of the group comprising: a compound; a biological structure, such as a cell or a cell fragment; an enzyme; and a chemical, all of which are found within the stomach of a patient; and gastric enzyme; enzyme substrate; gastric lipase; gastric hormone; pepsin; pepsinogen; intrinsic factor (IF); vitamin B12-IF complex; mucin; gastrin; and trypsin.

5. The in-line tester (10) of claim 4, wherein the target substance comprises gastric lipase and wherein the colorimetric substance comprises tributyrin, which detects metabolism of tributyrin by the colour change induced by production of butyric acid.

6. The in-line tester (10) of any preceding claim, comprising means for detecting the presence of two or more stomach-related markers, wherein the means for detecting the presence of two or more stomach-related markers are collocated on or in the first tester, wherein the first tester (52) comprises a liquid-absorbent layer (48), which is divided into discrete regions, each discrete region comprising a different colorimetric substance adapted to exhibit a colour change in the presence of different target substances.

7. The in-line tester (10) of claim 6, wherein the liquid-absorbent layer (48) comprises a first colorimetric substance adapted to exhibit a colour change, in use, according to the acidity/alkalinity of an aspirate drawn into the in-line tester; and further comprising a region of the liquid-absorbent layer comprising a second colorimetric substance adapted to produce an acid in the presence of a target stomach-related marker.

8. The in-line tester (10) of claim 7, wherein the liquid-absorbent layer (48) comprises pH indicator paper, and wherein a region of the pH indicator paper is coated, or impregnated, with tributyrin, wherein in the presence of gastric lipase, the tributyrin produces alcohol and butyric acid, the butyric acid being able to interact with the pH indicator paper to cause its colour to correspond to an acidic pH reading.

9. The in-line tester (10) of any preceding claim, wherein the colorimetric capnometer (64) comprises a paper substrate coated, or impregnated, with a dye that changes colour in the presence of carbon dioxide, wherein the dye optionally changes colour in the presence of greater than 5% carbon dioxide.

10. The in-line tester (10) of any preceding claim, further comprising a one-way valve (130) at, or downstream of, the outlet (33), wherein the cracking pressure for a given gas of the one-way valve (130) is less than the pressure required to force the same gas through the liquid-stop device (48/50), such that when a positive gas pressure is applied at the outlet (33), gas is preferentially vented through the one-way valve (130), rather than back through the in-line tester (10).

11. The in-line tester (10) of any preceding claim, comprising a main body comprising two parts that fit together to form the chamber, the two parts each having a mutually cooperating mating surface (166), which when placed against the mating surface of the other part, forms a fluid-tight seal, wherein one or both of the two parts comprises recesses, grooves or other voids (112) formed in their mating surface, the recesses, grooves or other voids forming internal chambers and fluid passageways of the in-line tester (10).

12. The in-line tester (10) of claim 11, further comprising an insert at least partially located within a chamber, the insert comprising an inlet which supports the first tester and a first vent aperture, the first vent aperture being arranged to direct a flow of gas passing through the in-line tester over the colorimetric capnometer.

13. The in-line tester (10) of any preceding claim, wherein the first (52) or second tester (64) and the liquid-stop device are in contact with one another, or are conjoined to form a laminated structure.

14. The in-line tester (10) of any preceding claim, wherein the liquid-stop device comprises a hydrophobic layer (50) comprising pores or perforations that enable, when dry, air to pass through them but when wetted by liquid, the liquid is repelled from the surface of the hydrophobic layer (50) and forced to overlie the openings of the pores or perforations, the pores or perforations of the hydrophobic layer (50) being smaller than the size of a liquid droplet.

15. The in-line tester (10) of any preceding claim, wherein the liquid-stop device permits the passage of water vapour therethrough, but prevents or inhibits the passage of liquid drops therethrough.

## Patentansprüche

1. Ein Inline-Prüfgerät (10), das Folgendes beinhaltet:
einen Einlass (38), der im Gebrauch mit einem Aspiratorschlauch (18) verbunden werden kann;
einen Auslass (33), der im Gebrauch mit einer Vakuumquelle verbunden werden kann; und
eine zwischen dem Einlass (38) und dem Auslass (33) liegende Kammer (14), wobei die Kammer (14) Folgendes beinhaltet:
ein erstes Prüfgerät (48), das eine erste kolorimetrische Substanz beinhaltet, die zum Aufzeigen einer Farbänderung in Gegenwart einer Zielsubstanz angepasst ist, die so angeordnet ist, dass von der Vakuumquelle in die Kammer (14) gezogene Fluide in Kontakt damit kommen;
wobei das Inline-Prüfgerät (10) ferner eine Flüssigkeits-Sperrvorrichtung (52) beinhaltet, die, wenn sie trocken ist, den Durchtritt von Gas von dem Einlass (38) zu dem Auslass (33) gestattet, die aber, wenn sie von einer Flüssigkeit benetzt wird, den Durchtritt von Fluiden von dem Einlass (38) zu dem Auslass (33) hemmt oder verhindert;
und **dadurch gekennzeichnet, dass**
die Kammer (14) ferner Folgendes beinhaltet:
ein zweites Prüfgerät (64), das ein kolorimetrisches Kapnometer beinhaltet, das in Gegenwart eines Kohlendioxids eine Farbänderung zeigt, das so angeordnet ist, dass von der Vakuumquelle in die Kammer (14) gezogene Fluide durch dieses hindurchtreten;
ein zwischen dem ersten Prüfgerät (48) und dem zweiten Prüfgerät (64) liegendes Reservoir;
wobei sich das zweite Prüfgerät (64) stromabwärts von der Flüssigkeits-Sperrvorrichtung (52) und vor dem Auslass (33) befindet.

2. Inline-Prüfgerät (10) gemäß Anspruch 1, wobei die Zielsubstanz eine Säure beinhaltet und wobei die kolorimetrische Substanz einen Säure-Base-Indikator beinhaltet.

3. Inline-Prüfgerät (10) gemäß Anspruch 2, wobei der Säure-Base-Indikator Lackmuspapier oder Bromthymolsulfonphthalein beinhaltet.

4. Inline-Prüfgerät (10) gemäß einem der vorhergehenden Ansprüche, wobei die Zielsubstanz einen auf den Magen bezogenen Marker beinhaltet, der eines oder mehrere aus der Gruppe sind, die Folgendes beinhaltet: eine Verbindung; eine biologische Struktur wie etwa eine Zelle oder ein Zellfragment; ein Enzym; und eine Chemikalie, die alle innerhalb des Magens eines Patienten zu finden sind; und Magenenzym; Enzymsubstrat; Magenlipase; Magenhormon; Pepsin; Pepsinogen; intrinsischer Faktor (IF); Vitamin B12-IF-Komplex; Muzin; Gastrin; und Trypsin.

5. Inline-Prüfgerät (10) gemäß Anspruch 4, wobei die Zielsubstanz Magenlipase beinhaltet und wobei die kolorimetrische Substanz Tributyrin beinhaltet, das den Tributyrinstoffwechsel durch die Farbänderung nachweist, die durch die Produktion von Buttersäure ausgelöst wird.

6. Inline-Prüfgerät (10) gemäß einem der vorhergehenden Ansprüche, das Mittel zum Nachweis des Vorhandenseins von zwei oder mehreren auf den Magen bezogenen Markern beinhaltet, wobei die Mittel zum Nachweis des Vorhandenseins von zwei oder mehreren auf den Magen bezogenen Markern auf oder in dem ersten Prüfgerät angeordnet sind, wobei das erste Prüfgerät (52) eine flüssigkeitsabsorbierende Schicht (48) beinhaltet, die in diskrete Regionen aufgeteilt ist, wobei jede diskrete Region eine unterschiedliche kolorimetrische Substanz beinhaltet, die zum Aufzeigen einer Farbänderung in Gegenwart unterschiedlicher Zielsubstanzen angepasst ist.

7. Inline-Prüfgerät (10) gemäß Anspruch 6, wobei die flüssigkeitsabsorbierende Schicht (48) eine erste kolorimetrische Substanz beinhaltet, die zum Aufzeigen einer Farbänderung im Gebrauch gemäß der Azidität/Alkalität eines in das Inline-Prüfgerät hineingezogenen Aspirats angepasst ist; und ferner eine Region der flüssigkeitsabsorbierenden Schicht beinhaltet, die eine zweite kolorimetrische Substanz beinhaltet, die zum Produzieren einer Säure in Gegenwart eines auf den Magen bezogenen Zielmarkers angepasst ist.

8. Inline-Prüfgerät (10) gemäß Anspruch 7, wobei die flüssigkeitsabsorbierende Schicht (48) pH-Indikatorpapier beinhaltet und wobei eine Region des pH-Indikatorpapiers mit Tributyrin beschichtet oder getränkt ist, wobei das Tributyrin in Gegenwart von Magenlipase Alkohol und Buttersäure produziert, wobei die Buttersäure in der Lage ist, mit dem pH-Indikatorpapier in Wechselwirkung zu treten, um zu bewirken, dass seine Farbe einem sauren pH-Messwert entspricht.

9. Inline-Prüfgerät (10) gemäß einem der vorhergehenden Ansprüche, wobei das kolorimetrische Kapnometer (64) ein Papiersubstrat beinhaltet, das mit einem Farbstoff beschichtet oder imprägniert ist, der in Gegenwart von Kohlendioxid die Farbe ändert, wobei der Farbstoff optional die Farbe in Gegenwart von mehr als 5% Kohlendioxid ändert.

10. Inline-Prüfgerät (10) gemäß einem der vorhergehenden Ansprüche, das ferner ein Einwegventil (130) an dem oder stromabwärts von dem Auslass (33) beinhaltet, wobei der Abreißdruck für ein gegebenes Gas des Einwegventils (130) geringer ist als der Druck, der erforderlich ist, um das gleiche Gas durch die Flüssigkeits-Sperrvorrichtung (48/50) zu drängen, sodass, wenn ein positiver Gasdruck an dem Auslass (33) angewendet wird, das Gas bevorzugt durch das Einwegventil abgelassen wird (130), anstatt zurück durch das Inline-Prüfgerät (10) zu strömen.

11. Inline-Prüfgerät (10) gemäß einem der vorhergehenden Ansprüche, das einen Hauptteil beinhaltet, der zwei Teile beinhaltet, die zusammenpassen, um die Kammer zu bilden, wobei die zwei Teile jeweils eine gegenseitig zusammenarbeitende Passfläche (166) aufweisen, die, wenn sie gegen die Passfläche des anderen Teils gelegt wird, eine fluiddichte Dichtung bildet, wobei einer der Teile oder beide Teile Aussparungen, Einkerbungen oder andere Leerräume (112) beinhalten, die in ihrer Passfläche ausgebildet sind, wobei die Aussparungen, Einkerbungen oder anderen Leerräume Innenkammern und Fluiddurchtrittskanäle des Inline-Prüfgeräts (10) bilden.

12. Inline-Prüfgerät (10) gemäß Anspruch 11, das ferner einen Einsatz beinhaltet, der sich mindestens teilweise innerhalb einer Kammer befindet, wobei der Einsatz einen Einlass beinhaltet, der das erste Prüfgerät und eine erste Entlüftungsöffnung stützt, wobei die erste Entlüftungsöffnung so angeordnet ist, dass sie einen durch das Inline-Prüfgerät durchtretenden Gasstrom über das kolorimetrische Kapnometer leitet.

13. Inline-Prüfgerät (10) gemäß einem der vorhergehenden Ansprüche, wobei das erste (52) oder zweite Prüfgerät (64) und die Flüssigkeits-Sperrvorrichtung in Kontakt miteinander stehen oder miteinander verbunden sind, um eine laminierte Struktur zu bilden.

14. Inline-Prüfgerät (10) gemäß einem der vorhergehenden Ansprüche, wobei die Flüssigkeits-Sperrvorrichtung eine hydrophobe Schicht (50) beinhaltet, die Poren oder Perforationen beinhaltet, die, wenn sie trocken sind, Luft durch sie hindurchtreten lassen, aber wenn sie mit Flüssigkeit benetzt werden, die Flüssigkeit von der Oberfläche der hydrophoben Schicht (50) abgestoßen wird und gezwungen wird, die Öffnungen der Poren oder Perforationen zu überlagern, wobei die Poren oder Perforationen der hydrophoben Schicht (50) kleiner sind als die Größe eines Flüssigkeitströpfchens.

15. Inline-Prüfgerät (10) gemäß einem der vorhergehenden Ansprüche, wobei die Flüssigkeits-Sperrvorrichtung den Durchtritt von Wasserdampf dadurch gestattet, aber den Durchtritt von Flüssigkeitstropfen dadurch hemmt oder verhindert.

## Revendications

1. Testeur en ligne (10) comprenant :
une entrée (38), pouvant être connectée, lors de l'utilisation, à un tube d'aspirateur (18) ;
une sortie (33) pouvant être connectée, lors de l'utilisation, à une source de vide ; et
une chambre (14) intercalée entre l'entrée (38) et la sortie (33), la chambre (14) comprenant :
un premier testeur (48) comprenant une première substance colorimétrique conçue pour présenter un changement de couleur en présence d'une substance cible, agencé de telle sorte que les fluides aspirés à l'intérieur de la chambre (14) par la source de vide viennent en contact avec lui ;
le testeur en ligne (10) comprenant en outre un dispositif d'arrêt de liquide (52) qui, lorsqu'il est sec, permet le passage de gaz de l'entrée (38) à la sortie (33), mais, lorsqu'il est mouillé par un liquide, inhibe ou empêche le passage de fluide de l'entrée (38) à la sortie (33) ;
et **caractérisé par**,
la chambre (14) comprenant en outre
un second testeur (64) comprenant un capnomètre colorimétrique, lequel présente un changement de couleur en présence de dioxyde de carbone, agencé de telle sorte que les fluides aspirés dans la chambre (14) par la source de vide passe à travers lui ;
un réservoir intercalé entre le premier testeur (48) et le second testeur (64) ;
le second testeur (64) étant situé en aval du dispositif d'arrêt de liquide (52) et avant la sortie (33).

2. Testeur en ligne (10) selon la revendication 1, dans lequel la substance cible comprend un acide, et dans lequel la substance colorimétrique comprend un indicateur acide-base.

3. Testeur en ligne (10) selon la revendication 2, dans lequel l'indicateur acide-base comprend du papier de tournesol ou du bleu de bromothymol.

4. Testeur en ligne (10) selon n'importe quelle revendication précédente, dans lequel la substance cible comprend un marqueur de type gastrique à savoir n'importe lequel ou lesquels des marqueurs du groupe comprenant : un composé ; une structure biologique, telle qu'une cellule ou un fragment cellulaire ; un enzyme, et une substance chimique, lesquels se trouvent tous à l'intérieur de l'estomac d'un patient ; et une enzyme gastrique; un substrat enzymatique, la lipase gastrique, une hormone gastrique, la pepsine, le pepsinogène, le facteur intrinsèque (IF) ; le complexe de vitamine B12-IF; une mucine ; la gastrine; et la trypsine.

5. Testeur en ligne (10) selon la revendication 4, dans lequel la substance cible comprend la lipase gastrique et dans lequel la substance colorimétrique comprend la tributyrine, lequel détecte un métabolisme de tributyrine par le changement de couleur induit par la production d'acide butyrique.

6. Testeur en ligne (10) selon n'importe quelle revendication précédente, comprenant des moyens de détection de la présence de deux ou plusieurs marqueurs de type gastrique, dans lequel les moyens de détection de la présence de deux ou plusieurs marqueurs de type gastrique sont cositués sur ou dans le premier testeur, dans lequel le premier testeur (52) comprend une couche absorbant les liquides (48), divisée en régions discrètes, chaque région discrète comprenant une substance colorimétrique différente conçue pour présenter un changement de couleur en présence de différentes substances cibles.

7. Testeur en ligne (10) selon la revendication 6, dans lequel la couche absorbant les liquides (48) comprend une première substance colorimétrique adaptée pour présenter un changement de couleur, lors de l'utilisation, en fonction de l'acidité/alcalinité d'un aspirat aspiré dans le testeur en ligne ; et comprenant en outre une région de la couche absorbant les liquides qui comprend une seconde substance colorimétrique conçue pour produire un acide en présence d'un marqueur de type gastrique cible.

8. Testeur en ligne (10) selon la revendication 7, dans lequel la couche absorbant les liquides (48) comprend un papier indicateur de pH, et dans lequel une région du papier indicateur de pH est revêtue, ou imprégnée, de tributyrine, dans lequel en présence de lipase gastrique, la tributyrine produit de l'alcool et de l'acide butyrique, l'acide butyrique pouvant interagir avec le papier indicateur de pH pour faire en sorte que sa couleur corresponde à une lecture de pH acide.

9. Testeur en ligne (10) selon n'importe quelle revendication précédente, dans lequel le capnomètre colorimétrique (64) comprend un substrat de papier revêtu, ou imprégné, d'une teinture qui change de couleur en présence de dioxyde de carbone, dans lequel la teinture change facultativement de couleur en présence de plus de 5 % de dioxyde de carbone.

10. Testeur en ligne (10) selon n'importe quelle revendication précédente, comprenant en outre un clapet anti-retour (130) au niveau, ou en aval, de la sortie (33), dans lequel la pression de craquage pour un gaz donné du clapet anti-retour (130) est inférieure à la pression requise pour forcer le même gaz à travers le dispositif d'arrêt de liquide (48/50), de telle sorte que lorsqu'une pression de gaz positive est appliquée au niveau de la sortie (33), le gaz soit de préférence éventé à travers le clapet anti-retour (130), plutôt que de retour à travers le testeur en ligne (10).

11. Testeur en ligne (10) selon n'importe quelle revendication précédente, comprenant un corps principal comprenant deux parties qui s'enclenchent l'une avec l'autre pour former la chambre, les deux parties comportant chacune une surface d'accouplement mutuellement coopérative (166), laquelle, à son placement contre la surface d'accouplement de l'autre partie, forme un joint étanche aux fluides, dans lequel une des deux parties ou les deux comprennent des évidements, des rainures ou d'autres vides (112) formés dans leur surface d'accouplement, les évidements, rainures ou autres vides formant des chambres internes et des passages de fluide du testeur en ligne (10).

12. Testeur en ligne (10) selon la revendication 11, comprenant en outre un insert situé au moins partiellement à l'intérieur d'une chambre, l'insert comprenant une entrée qui soutient le premier testeur et une première ouverture d'évent, la première ouverture d'évent étant agencée pour diriger un flux de gaz traversant le testeur en ligne par-dessus le capnomètre colorimétrique.

13. Testeur en ligne (10) selon n'importe quelle revendication précédente, dans lequel le premier (52) ou le second testeur (64) et le dispositif d'arrêt de liquide sont en contact l'un avec l'autre, ou sont conjoints pour former une structure stratifiée.

14. Testeur en ligne (10) selon n'importe quelle revendication précédente, dans lequel le dispositif d'arrêt de liquide comprend une couche hydrophobe (50) comprenant des pores ou des perforations qui permettent, lorsqu'ils sont secs, le passage d'air à travers eux, mais qui, lorsqu'ils sont mouillés par un liquide, repoussent le liquide de la surface de la couche hydrophobe (50), lequel est alors forcé à reposer par-dessus les ouvertures des pores ou perforations, les pores ou perforations de la couche hydrophobe (50) étant plus petits que la taille d'une gouttelette de liquide.

15. Testeur en ligne (10) selon n'importe quelle revendication précédente, dans lequel le dispositif d'arrêt de liquide laisse passer la vapeur d'eau à travers lui, mais empêche ou inhibe le passage de gouttelettes de liquide à travers lui.
